(19) Europäisches Patentamt
European Patent Office
Office européen des brevets

(11) Veröffentlichungsnummer: 0 001 813
B1

(12) # EUROPÄISCHE PATENTSCHRIFT

(45) Veröffentlichungstag der Patentschrift: 04.11.81

(21) Anmeldenummer: 78101244.8

(22) Anmeldetag: 27.10.78

(51) Int. Cl.³: C 07 D 233/72, A 61 K 31/415, C 07 C 127/19, C 07 D 233/88

(54) **Imidazolidinderivate, Verfahren zu deren Herstellung, Zwischenprodukte zur Verwendung in diesem Verfahren, entsprechende pharmazeutische Präparate sowie die Verwendung der Wirkstoffe.**

(30) Priorität: 28.10.77 LU 78407
19.05.78 CH 5465/78
06.09.78 CH 9367/78

(43) Veröffentlichungstag der Anmeldung: 16.05.79 Patentblatt 79/10

(45) Bekanntmachung des Hinweises auf die Patenterteilung: 04.11.81 Patentblatt 81/44

(84) Benannte Vertragsstaaten: BE CH DE FR LU NL SE

(56) Entgegenhaltungen:
BE - A - 629 779
DE - A - 1 915 689
DE - A - 1 958 183
DE - A - 2 649 925
US - A - 3 676 456

(73) Patentinhaber: F. HOFFMANN-LA ROCHE & CO. Aktiengesellschaft
CH-4002 Basel (CH)

(72) Erfinder: Bernauer, Karl, Prof.Dr.
Wartenbergstrasse
CH-4104 Oberwil (CH)
Erfinder: Link, Helmut, Dr.
Dammerkirchstrasse
CH-4056 Basel (CH)
Erfinder: Stohler, Harro, Dr.
Im Katzenwadel 12
CH-4102 Binningen (CH)

(74) Vertreter: Lederer, Franz, Dr. et al,
Patentanwälte Lederer, Meyer Lucile-Grahn-Strasse 22
D-8000 München 80 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Imidazolidinderivate, Verfahren zu deren Herstellung, Zwischenprodukte zur Verwendung in diesem Verfahren, entsprechende pharmazeutische Präparate sowie die Verwendung der Wirkstoffe

Die vorliegende Erfindung betrifft neue Imidazolidinderivate der allgemeinen Formel

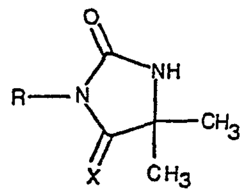

I

in der X Sauerstoff oder Imino und R eine der Gruppen

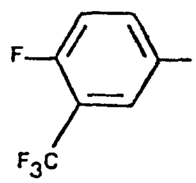 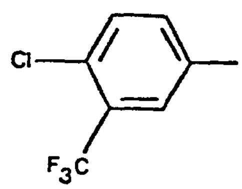 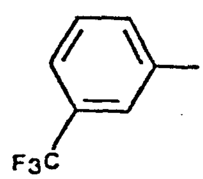 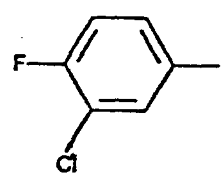 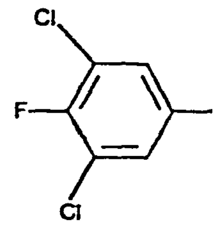

darstellt.

Es wurde gefunden, dass die neuen erfindungsgemässen Imidazolidinderivate der Formel I wertvolle therapeutische Eigenschaften besitzen; sie können demnach als Arzneimittel Verwendung finden. Beispielsweise zeichnen sie sich durch antiandrogene Wirkung aus und können demgemäss als antiandrogen wirkende Mittel verwendet werden, insbesondere zur Behandlung von Krankheiten, die mit einer erhöhten androgenen Aktivität in Verbindung stehen, wie z.B. Akne, Seborrhoe, Hirsutismus und Prostata-adenom. Besonders bevorzugte, antiandrogen wirksame Verbindungen der Formel I sind diejenigen, worin X Sauerstoff darstellt; darunter besonders bevorzugt ist 3-(3-Chlor-4-fluor-phenyl)-5,5-dimethyl-hydantoin. Diejenigen der Imidazolidinderivate der Formel I, worin R eine der ersten vier der obigen Gruppen bedeutet, sind schistosomicid wirksam und können somit zur Therapie und Verhütung der Bilharziose eingesetzt werden. Bevorzugte Verbindungen sind diejenigen, worin X Sauerstoff darstellt. Ganz besonders bevorzugt wegen ihrer starken schistosomiciden Wirkung sind 3-(3-Trifluormethyl-4-fluor-phenyl)-5,5-dimethyl-hydantoin und 1-(3-Trifluormethyl-4-fluor-phenyl)-5-imino-4,4-dimethyl-2-imidazolidinon.

In der DE-A 2,649,925 sind Imidazolidinderivate mit einer ähnlichen Struktur beschrieben, jedoch besitzen diese Verbindungen im Phenylring in der 4-Stellung eine Nitrogruppe. Die erfindungsgemässen Verbindungen unterscheiden sich strukturell signifikant von diesen vorbekannten Verbindungen und weisen zum grössten Teil auch ein anderes Wirkungsmuster auf.

Die Imidazolidinderivate der Formel I können erfindungsgemäss dadurch hergestellt werden, dass man

a) zur Herstellung einer Verbindung der Formel I, in der X Sauerstoff darstellt, eine Verbindung der allgemeinen Formel

$$R-NH-CO-NH-\underset{\displaystyle CH_3}{\overset{\displaystyle CH_3}{\underset{|}{\overset{|}{C}}}}-COY \qquad II$$

in der R die obige Bedeutung hat und Y die Gruppe $-OR^1$ oder $-NR^2R^3$ darstellt, worin $R^1$ Wasserstoff oder niederes Alkyl und $R^2$ und $R^3$ beide niederes Alkyl darstellen,
cyclisiert, oder dass man

b) zur Herstellung einer Verbindung der Formel I, in der X Imino bedeutet, eine Verbindung der allgemeinen Formel

$$R-NCO \qquad III$$

in der R die obige Bedeutung hat,
unter wasserfreien Bedingungen mit $\alpha$-Amino-isobutyronitril umsetzt, oder dass man

c) zur Herstellung einer Verbindung der Formel I, in der X Sauerstoff darstellt, eine Verbindung der allgemeine Formel

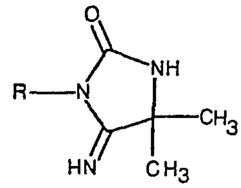

Ia

in der R die obige Bedeutung hat,
einer Hydrolyse unterwirft.

Diejenigen der neuen Ausgangsverbindungen der Formel II, welche Säureamide darstellen, sind ebenfalls Gegenstand der Erfindung.

Nachstehend werden die verschiedenen Ausführungsformen des erfindungsgemässen Verfahrens zur Herstellung der Imidazolidinderivate der Formel I näher erläutert.

Ausgangsamide der Formel II, d.h. Verbindungen II mit $Y = NR^2R^3$, können durch Umsetzung einer substituierten Benzhydroxamsäure der allgemeinen Formel

R—CONHOH IV

in der R die oben gegebene Bedeutung hat,
mit einem Azirinderivat der allgemeinen Formel

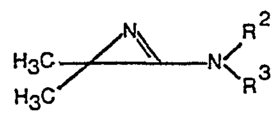

V

in der $R^2$ und $R^3$ die oben gegebene Bedeutung haben,
hergestellt werden. Die Reaktion gelingt in einem inerten organischen Lösungsmittel, wie Tetrahydrofuran, Aether oder Dioxan, bei Zimmertemperatur und unter leichtem Erwärmen, z.B. auf 50°C.

Die erwähnten Ausgangsamide der Formel II, welche ebenfalls Gegenstand der Erfindung sind, leiten ihre erfinderische Qualität von dem Umstand ab, dass sie gemäss der obigen Verfahrensalternative a) in die therapeutisch wertvollen Imidazolidinderivate der Formel I übergeführt werden können. Zwar sind aus US-A-3,676,456, DE-A-1,915,689 und DE-A-1,958,183 ähnliche Verbindungen bekannt, welche ebenfalls Zwischenprodukte zur Herstellung von Imidazolidinderivaten sind. Diese Imidazolidinderivate sind jedoch von den vorliegenden Imidazolidinderivaten der Formel I strukturell verschieden.

Die Herstellung der Ausgangsester der Formel II, d.h. Verbindungen II mit $Y = OR^{10}$, worin $R^{10}$ niederes Alkyl bedeutet, darstellt, kann durch Umsetzung einer Verbindung der allgemeinen Formel

$R—Z_1$ VI

unter wasserfreien Bedingungen mit einer Verbindung der allgemeinen Formel

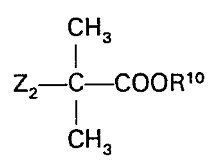

VII

worin R und $R^{10}$ die obige Bedeutung haben und eines der Symbole $Z_1$ und $Z_2$ Amino und das andere die Isocyanatgruppe —NCO darstellt,
erfolgen. Diese Additionsreaktion kann ohne Lösungsmittelzusatz, d.h. mittels einer Schmelze, oder auch durch Erwärmung in einem inerten, wasserfreien Lösungsmittel, z.B. Tetrahydrofuran, Aether, Dioxan, Benzol oder Toluol, durchgeführt werden. Die Temperatur für die Umsetzung liegt vorzugsweise im Bereiche von etwa 0—120°C. Beim Arbeiten mit einer Schmelze ist darauf zu achten, dass höhere Temperaturen bzw. längere Reaktionszeiten zur Cyclisation unter Bildung des entsprechenden Hydantoins der Formel I führt. Zur Isolierung eines Ausgangsesters II soll die Reaktion deshalb rechtzeitig unterbrochen werden, was beispielsweise durch dünnschichtchromatographische Kontrolle bewerkstelligt werden kann. Es ist jedoch bevorzugt, den Ester der Formel II nicht zu isolieren, sondern die Reaktion bis zur Bildung des entsprechenden Hydantoins der Formel I laufen zu lassen (wie nachstehend ausgeführt).

Zur Herstellung einer Carbonsäure der Formel II, d.h. einer Verbindung II mit Y = Hydroxy, kann man ein Amid der Formel II in einem inerten organischen Lösungsmittel, wie z.B. Methanol oder Aethanol, lösen und mit wässrigem Alkali, z.B. Natron- oder Kalilauge, bei einer Temperatur zwischen der Raumtemperatur und dem Siedepunkt des Reaktionsgemisches behandeln. Die entsprechende Carbonsäure der Formel II wird in üblicher Weise durch Neutralisation des gebildeten Carbonsäuresalzes, beispielsweise mit einer Mineralsäure, erhalten.

Die erfindungsgemässe Cyclisation der Ausgangsverbindungen der Formel II führt zu Hydantoinen der Formel I, d.h. Verbindungen I mit X = Sauerstoff. Diese Cyclisation erfolgt z.B. unter den Bedingungen einer sauren Hydrolyse, vorzugsweise durch Behandeln mit einer wässrigen Mineralsäure, wie wässriger Salzsäure. Die Hydrolyse von Amiden der Formel II erfolgt vorteilhaft in einem inerten organischen Lösungsmittel, z.B. Tetrahydrofuran, oder Dioxan, und bei etwa raumtemperatur, wobei auch bei höheren Temperaturen, z.B. bis zur Siedetemperatur des Reaktionsgemisches, gearbeitet werden kann. Der Ringschluss von Estern oder Carbonsäuren der Formel II erfolgt bevorzugt mit einem mit Wasser mischbaren Lösungsmittel, wie Aceton, Methyläthylketon, Tetrahydrofuran, Dimethoxyäthan oder Dioxan. Ein bevorzugtes Cyclisierungsmittel ist 6-n wässrige Salzsäure und Aceton in einem Gewichtsverhältnis von etwa 1:1. Die Reaktionstemperatur liegt vorzugsweise bei der Siedetemperatur des Reaktionsgemisches, wobei auch niedrigere Reaktionstemperaturen, z.B. bis Raumtemperatur, mit entsprechend längeren Reaktionszeiten angewendet werden können. Ausgangsester der Formel II können ebenfalls durch Erwärmen ohne Lösungsmittel, d.h. mittels einer Schmelze, cyclisiert werden. Die Temperatur für diese Schmelzreaktion liegt vorzugsweise im Bereich von etwa 100—200°C, insbesondere im Bereiche von etwa 120—160°C. Nach einer bevorzugten Ausführungsform wird diese Cyclisierung durch Einsatz der für die Herstellung der Ausgangsester der Formel II verwendbaren Verbindungen der obigen Formeln VI und VII durchgeführt, wobei die Reaktion durch Wahl genügender Reaktionszeit bzw. Reaktionstemperatur ohne Isolierung der intermediär gebildeten Ester der Formel II bis zum gewünschten Cyclisationsprodukt, d.h. Hydantoin der Formel I, läuft. Die Reaktion kann beispielsweise dünnschichtchromatographisch verfolgt werden.

Die erfindungsgemässe wasserfreie Umsetzung des Isocyanats der Formel III mit $\alpha$-Aminoisobutyronitril führt zu Imidazolidinderivaten der Formel I, worin X Imino bedeutet. Die Reaktion kann unter den gleichen Bedingungen durchgeführt werden wie die oben beschreibene Cyclisation unter Verwendung von Verbindungen der Formeln VI und VII, d.h. mittels einer Schmelze. Es ist auch möglich, das Iminoderivat durch Erhitzen der Ausgangsverbindungen mit einem ätherischen Lösungsmittel, z.B. mit Dioxan oder Dimethoxyäthan, zu erhalten.

Die erfindungsgemässe Hydrolyse der Ausgangsverbindungen der Formel Ia führt zu entsprechenden Hydantoinen der Formel I, d.h. Verbindungen I mit X = Sauerstoff. Die Hydrolyse kann, gegebenenfalls unter Zusatz eines inerten Lösungsmittels, durch Behandeln z.B. mit einer Mineralsäure, wie Salzsäure, bei einer Temperatur zwischen der Raumtemperatur und dem Siedepunkt des Reaktionsgemisches durchgeführt werden.

Die Verbindungen der Formel I sind zum Teil kristalline, feste Substanzen, die in niederen Alkanolen, wie Methanol oder Aethanol, in Dimethylsulfoxid, Dimethylformamid und Hexamethylphosphorsäuretriamid und z.T. auch in chlorierten Kohlenwasserstoffen, wie Chloroform, Methylenchlorid und Tetrachlorkohlenstoff, relativ gut löslich und in Aether, Benzol, und Wasser relativ schwerlöslich sind.

Wie gesagt, besitzen die erfindungsgemässen Endprodukte der Formel I antiandrogene Wirkung. Diese Wirkung kann z.B. durch folgenden Versuch nachgewiesen werden:

Einer Gruppe von 5 männlichen, sterilisierten Ratten werden täglich während 7 Tagen jeweils 10 mg des zu untersuchenden Präparates pro kg zusammen mit 0,5 mg Testosteronpropionat pro kg s.c. verabreicht. 2 Kontrollgruppen zu jeweils 5 Ratten erhalten jeweils keine Behandlung bzw. nur Testosteronpropionat. Die Gewichtsabnahme der ventralen Prostata und der Samenblase gibt ein Mass für die antiandrogene Wirkung.

Die nachstehend Tabelle I gibt über einige Versuchsergebnisse Aufschluss.

TABELLE I

| Verbindung | Ventrale Prostata mg | Samenblase mg |
|---|---|---|
| Kontrolle | 7,6 ± 0,4 | 6,3 ± 0,5 |
| Testosteronpropionat | 69,0 ± 3,9 | 47,0 ± 3,1 |
| 3-(3-Trifluoromethyl-4-chlor-phenyl)-5,5-dimethyl-hydantoin + Testosteronpropionat | 25,0 ± 1,0 | 23,0 ± 1,6 |
| Kontrolle | 8,0 ± 0,4 | 6,3 ± 0,4 |
| Testosteronpropionat | 58,0 ± 2,0 | 42,0 ± 1,6 |
| 1-(3-Trifluoromethyl-4-chlor-phenyl)-5-imino-4,4-dimethyl-2-imidazolidinon + Testosteron-propionat | 29,0 ± 2,2 | 24,0 ± 0,6 |
| 3-(3-Trifluoromethyl-phenyl)-5,5-dimethyl-hydantoin + Testosteronpropionat | 35,0 ± 1,9 | 29,0 ± 1,4 |
| 3-(3-Trifluoromethyl-4-fluor-phenyl)-5,5-dimethyl-hydantoin + Testosteronpropionat | 21,0 ± 1,0 | 18,0 ± 0,9 |
| Kontrolle | 14 ± 1 | 24 ± 2 |
| Testosteronpropionat | 121 ± 13 | 100 ± 9 |
| 1-(3-Trifluoromethyl-4-fluor-phenyl)-5-imino-4,4-dimethyl-2-imidazolidinon + Testosteronpropionat | 31 ± 4 | 30 ± 2 |
| Kontrolle | 22 ± 2 | 48 ± 4 |
| Testosteronpropionat | 184 ± 8 | 145 ± 7 |
| 3-(3,5-Dichlor-4-fluorphenyl)-5,5-dimethyl-hydantoin-Testosteronpropionat | 72 ± 9 | 62 ± 3 |

Die schistosomicide Wirkung der oben definierten erfindungsgemässen Endprodukte kann durch folgenden Versuch nachgewiesen werden:

Mäuse werden subcutan mit 60 Cercarien von Schistosoma mansoni infiziert. Ca. 42 Tage nach der Infektion werden die Tiere einmal bzw. (in einem weiteren Versuch) an 5 aufeinanderfolgenden Tagen mit den zu prüfenden Verbindungen peroral behandelt. Pro Verbindung und Dosierung (mg/kg) werden 5—10 Tiere eingesetzt. Als Kontrolle dienen 10 unbehandelte Tiere. Die Sektion erfolgt 6 Tage bzw. 2—3 Wochen nach Behandlungsabschluss. Wurmpaare in Mesenterialvenen, Pfortader und Leber werden herauspräpariert und gezählt. Die prozentuale Verteilung der Wurmpaare in Mesenterialvenen, Pfortader und Leber wird berechnet und der Zustand der Würmer (lebend, tot) registriert. Die Präparatwirkung zeigt sich in einem erhöhten Anteil der Würmer in den Gefässen der Leber und im Auftreten toter Würmer.

Zur Auswertung vergleicht man den prozentualen Anteil lebender und toter Wurmpaare in den

Gefässen der Leber sowohl bei infizierten, behandelten Tieren als auch bei infizierten aber unbehandelten Kontrolltieren. Die Bestimmung der $VD_{50}$ (Vermizide Dosis 50%: die Dosis, die 50% der Wurmpaare abtötet) erfolgt nach der Probitmethode.

Einige Versuchsergebnisse sind in der nachstehenden Tabelle II zusammengefasst:

TABELLE II

| Verbindung | $VD_{50}$ mg/kg p.o. | |
|---|---|---|
| | 1 mal dosiert | 5 mal dosiert |
| 3-(3-Trifluormethyl-4-fluor-phenyl)-5,5-dimethyl-hydantoin | 47 | 15 |
| 3-(3-Trifluormethyl-4-chlor-phenyl)-5,5-dimethyl-hydantoin | 59 | 29 |
| 3-(3-Trifluormethyl-phenyl)-5,5-dimethyl-hydantoin | 60 | 32 |
| 3-(3-Chlor-4-fluor-phenyl)-5,5-dimethyl-hydantoin | | 68 |
| 1-(3-Trifluormethyl-4-chlorphenyl)-5-imino-4,4-dimethyl-2-imidazolidinon | 58 | 33 |
| 1-(3-Trifluormethyl-4-fluor-phenyl)-5-imino-4,4-dimethyl-2-imidazolidinon | 37 | 21 |

Die toxische Wirkung an der Maus (Beobachtung nach 24 Stunden) wurde ebenfalls ermittelt und geht aus der nachstehenden Tabelle III hervor:

TABELLE III

| Verbindung | $LD_{50}$ mg/kg p.o. |
|---|---|
| 3-(3-Trifluormethyl-4-fluor-phenyl)-5,5-dimethyl-hydantoin | 1250–2500 |
| 3-(3-Trifluormethyl-4-chlor-phenyl)-5,5-dimethyl-hydantoin | 5000 |
| 3-(3-Trifluormethyl-phenyl)-5,5-dimethyl-hydantoin | 625–1250 |
| 3-(3-Chlor-4-fluor-phenyl)-5,5-dimethyl-hydantoin | 2500–5000 |
| 1-(3-Trifluormethyl-4-chlorphenyl)-5-imino-4,4-dimethyl-2-imidazolidinon | 312–625 |
| 1-(3-Trifluormethyl-4-fluor-phenyl)-5-imino-4,4-dimethyl-2-imidazolidinon | 312–625 |
| 3-(3,5-Dichlor-4-fluor-phenyl)-5,5-dimethyl-hydantoin | 1250–2500 |

Die erfindungsgemässen Produkte können als Heilmittel, z.B. in Form pharmazeutischer Präparate Verwendung finden, welche sie in Mischung mit einem für die enterale oder parenterale Applikation geeigneten pharmazeutischen organischen oder anorganischen Trägermaterial, wie Gelatine, Milchzucker, Stärke, Gummi arabicum, Magnesiumstearat, Talcum, pflanzliche Oele, Polyalkylenglykole, Vaseline u.dgl. mehr enthalten. Die Präparate können in fester Form, z.B. als Tabletten, Dragées, oder in flüssiger Form, z.B. als Lösungen, Suspensionen oder Emulsionen, vorliegen. Sie können Hilfsstoffe, wie Konservierungs-, Stabilisierungs-, Netz- oder Emulgiermittel, Salze zur Veränderung des osmotischen Druckes oder Puffer enthalten. Auch andere therapeutisch wirksame Stoffe können beigemischt sein.

Zweckmässige pharmazeutische Dosierungsformen enthalten etwa 10—500 mg einer Verbindung der Formel I.

Die Dosierung wird entsprechend den individuellen Erfordernissen gewählt. Zum Beispiel können diese Verbindungen in Dosierungen von etwa 0,1 mg/kg bis etwa 50 mg/kg pro Tag p.o., verabreicht werden.

Als antiandrogene Mittel verwendbare Dosierungsformen enthalten zweckmässig etwa 10—500 mg, vorzugsweise etwa 100 mg Wirkstoff. Die Dosierung ist beispielsweise etwa 0,1 mg/kg bis etwa 10 mg/kg pro Tag p.o., vorzugsweise etwa 1 mg/kg pro Tag p.o. Zweckmässigerweise wird diese Dosis in Anpassung an den Zustand des Patienten etwa 3—8 Monate täglich verabreicht.

Als schistosomicide Mittel verwendbare Dosierungsformen enthalten zweckmässig etwa 100—500 mg, vorzugsweise etwa 250 mg Wirkstoff. Die Dosierung ist beispielsweise etwa 5 mg/kg bis etwa 50 mg/kg pro Tag p.o., vorzugsweise 25 mg/kg p.o. pro Tag. Diese Menge kann in einer einzelnen Dosierung oder in mehreren unterteilten Dosierungen verabreicht werden, je nach Bedürfnis des Patienten und Vorschrift des Fachmannes. Zweckmässigerweise wird diese Dosis in Anpassung an den Zustand des Patienten an einem oder an mehreren aufeinanderfolgenden Tagen verabreicht.

Die nachstehenden Beispiele illustrieren die Erfindung. Die Temperaturen sind in °C angegeben.

## Beispiel 1

14,5 g (100 mMol) 3-Chlor-4-fluoranilin und 17,2 g (120 mMol) 2-Isocyanato-2-methylpropionsäuremethylester werden bei 140° geschmolzen und 6 Stunden bei dieser Temperatur gehalten. Der intermediär gebildete N-[(3-Chlor-4-fluorphenyl)-carbamoyl]-2-methylalaninmethylester wird nicht isoliert. Das erhaltene 3-(3-Chlor-4-fluorphenyl)-5,5-dimethylhydantoin wird aus Isopropanol/Methanol/Methylenchlorid kristallisiert. Nach 16-stündigem Trocknen unter stark vermindertem Druck bei 55° schmilzt das Produkt bei 165—166°.

Analyse:
Berechnet für $C_{11}H_{10}ClFN_2O_2$ (256,66):

| | | | | |
|---|---|---|---|---|
| | C 51,48, | H 3,93, | N 10,91, | Cl 13,81% |
| Gefunden: | C 51,51, | H 3,78, | N 11,11, | Cl 13,92%. |

## Beispiel 2

In der in Beispiel 1 angegebenen Weise erhält man aus 3,5-Dichlor-4-fluor-anilin und 2-Isocyanato-2-methylpropionsäure-methylester und Kristallisieren aus Aether/Petroläther 3-(3,5-Dichlor-4-fluorphenyl)-5,5-dimethyl-hydantoin, das bei 175—176° schmilzt. Der intermediär gebildete N-[(3,5-Dichlor-4-fluorphenyl)-carbamoyl]-2-methylalaninmethylester wird nicht isoliert.

Analyse:
Berechnet für $C_{11}H_9Cl_2FN_2O_2$ (291,11):

| | | | | |
|---|---|---|---|---|
| | C 45,39, | H 3,12, | N 9,62, | Cl 24,36% |
| Gefunden: | C 45,55, | H 3,19, | N 9,57, | Cl 24,58%. |

In der gleichen Weise erhält man:
3-(3-Trifluormethyl-4-fluor-phenyl)-5,5-dimethyl-hydantoin; Smp. 111—112°.

## Beispiel 3

Einer Lösung von 8,00 g (23,6 mMol) N-[(3-Trifluormethyl-4-chlorphenyl)-carbamoyl]-2-methylalaninmethylester in 20 ml 6-n wässriger Salzsäure und 20 ml Aceton werden 2 Stunden auf dem Dampfbad erwärmt und anschliessend eingeengt. Das ausgefallene Produkt wird zuerst aus Methylenchlorid und anschliessend aus Methylenchlorid/Petroläther umkristallisiert. Nach 20 stündigem Trocknen unter stark vermindertem Druck bei 50° erhält man 3-(3-Trifluormethyl-4-chlorphenyl)-5,5-dimethylhydantoin vom Schmelzpunkt 152—153°.

Analyse:
Berechnet für $C_{12}H_{10}ClF_3N_2O_2$ (306,67):

| | | | |
|---|---|---|---|
| | C 47,00, | H 3,29, | N 9,13% |
| Gefunden: | C 46,89, | H 3,23, | N 8,93%. |

In der gleichen Weise erhält man:

3-(3-Trifluormethylphenyl)-5,5-dimethylhydantoin; Smp. 105—106°.

Der als Ausgangsverbindung eingesetzte N-[(3-Trifluormethyl-4-chlorphenyl)-carbamoyl]-2-methylalaninmethylester kann wie folgt hergestellt werden:

9,75 g (50 mMol) 5-Amino-2-chlorbenzotrifluorid und 7,15 g (50 mMol) 2-Isocyanato-2-methylpropionsäure-methyl-ester werden bei 80° geschmolzen und 30 Minuten bei dieser Temperatur gehalten. Das dabei auskristallisierte Produkt wird aus Isopropanol/Methylenchlorid umkristallisiert und 20 Stunden unter stark vermindertem Druck bei 50° getrocknet. Man erhält N-[(3-Trifluormethyl-4-chlorphenyl)-carbamoyl]-2-methylalaninmethylester, der bei 141—142° schmilzt.

Analyse:

| | | | |
|---|---|---|---|
| Berechnet für $C_{13}H_{14}ClF_3N_2O_3$ (338,71): | | | |
| | C 46,10, | H 4,17, | N 8,27% |
| Gefunden: | C 46,17, | H 4,14, | N 8,26%. |

Der als Ausgangsverbindung verwendete N-[(3-Trifluormethylphenyl)-carbamoyl]-2-methylalaninester kann in ähnlicher Weise hergestellt werden (Schmelze 120 Minuten bei 130°).

Beispiel 4

14,5 g (65 mMol) 3-Trifluormethyl-4-chlorphenylisocyanat und 5,5 g (65 mMol) $\alpha$-Amino-isobutyronitril werden 20 Minuten bei 100° gehalten. Das erhaltene dickflüssige Oel wird zur Reinigung an 500 g Kieselgel (Korngrösse etwa 0,06—0,2 mm) mit Methylenchlorid/Methanol chromatographiert. Das erhaltene Produkt wird aus Methylenchlorid/Petroläther kristallisiert. Nach 15-stündigem Trocknen unter stark vermindertem Druck bei 50° erhält man 1-(3-Trifluormethyl-4-chlorphenyl)-5-imino-4,4-dimethyl-2-imidazolidinon, das bei 148—149° schmilzt.

Analyse:

| | | | | |
|---|---|---|---|---|
| Berechnet für: $C_{12}H_{11}ClF_3N_3O$ (305,69): | | | | |
| | C 47,15, | H 3,63, | H 13,75, | Cl 11,60% |
| Gefunden: | C 47,11, | H 3,66, | N 13,84, | Cl 11,86%. |

In der gleichen Weise wird hergestellt:

1-(3-Trifluormethyl-4-fluor-phenyl)-5-imino-4,4-dimethyl-2-imidazolidinon; Smp. 92—93° (aus Aether/n-Pentan).

Analyse:

| | | | |
|---|---|---|---|
| Berechnet für $C_{12}H_{11}F_4N_3O$ (289,23): | | | |
| | C 49,83, | H 3,83, | N 14,53% |
| Gefunden: | C 49,69, | H 3,48, | N 14,18% |

Beispiel 5

61 mg (0,2 mMol) 1-(3-Trifluormethyl-4-chlorphenyl)-5-imino-4,4-dimethyl-2-imidazolidinon werden in 4 ml 0,1-n wässriger Salzsäure gelöst und mit 2 ml Wasser versetzt. Die allmählich ausfallenden weissen Nadeln werden nach 20 Stunden filtriert, mit Wasser gewaschen und 2 Stunden unter stark vermindertem Druck bei 65° getrocknet. Man erhält 3-(3-Trifluormethyl-4-chlorphenyl)-5,5-dimethylhydantoin, das bei 156—157,5° schmilzt.

Beispiel 6

Herstellung von Tabletten nachstehender Zusammensetzung:

| | |
|---|---|
| Wirkstoff der Formel I | 100,0 mg |
| Milchzucker | 40,0 mg |
| Maisstärke | 34,0 mg |
| Aethylcellulose | 4,0 mg |
| Talcum | 1,8 mg |
| Magnesiumstearat | 0,2 mg |
| | 180,0 mg |

Der Wirkstoff wird mit dem Milchzucker und der Maisstärke vermischt und mit einer Lösung der

Aethylcellulose in 16 ml Methylenchlorid granuliert. Das Granulat wird bei 40° getrocknet, mit dem Talcum und Magnesiumstearat vermischt und zu Tabletten gepresst.

| | |
|---|---|
| Gewicht einer Tablette | 180 mg |
| Wirkstoffgehalt einer Tablette | 100 mg |

Beispiel 7

Herstellung von Tabletten nachstehender Zusammensetzung:

| | |
|---|---|
| Wirkstoff der Formel I | 250,0 mg |
| Milchzucker | 100,0 mg |
| Maisstärke | 85,0 mg |
| Aethylcellulose | 10,0 mg |
| Talcum | 4,5 mg |
| Magnesiumstearat | 0,5 mg |
| | 450,0 mg |

Der Wirkstoff wird mit dem Milchzucker und der Maisstärke vermischt und mit einer Lösung der Aethylcellulose in 40 ml Methylenchlorid granuliert. Das Granulat wird bei 40° getrocknet, mit dem Talcum und Magnesiumstearat vermischt und zu Tabletten gepresst.

| | |
|---|---|
| Gewicht einer Tablette | 450 mg |
| Wirkstoffgehalt einer Tablette | 250 mg |

Beispiel 8

Herstellung von Kapseln nachstehender Zusammensetzung:

| | |
|---|---|
| Wirkstoff der Formel I | 100,0 mg |
| Milchzucker | 62,0 mg |
| Maisstärke | 12,0 mg |
| Talcum | 6,0 mg |
| | 180,0 mg |

Der Wirkstoff wird mit dem Milchzucker und der Maisstärke homogen vermischt, durch eine Siebmaschine passiert und nach Untermischen des Talcums in Gelatinekapseln abgefüllt

| | |
|---|---|
| Kapselfüllgewicht | 180 mg |
| Wirkstoffgehalt | 100 mg |

Beispiel 9

Herstellung von Kapseln nachstehender Zusammensetzung:

| | |
|---|---|
| Wirkstoff der Formel I | 250,0 mg |
| Milchzucker | 155,0 mg |
| Maisstärke | 30,0 mg |
| Talcum | 15,0 mg |
| | 450,0 mg |

Der Wirkstoff wird mit dem Milchzucker und der Maisstärke homogen vermischt, durch eine Siebmaschine passiert und nach Untermischen des Talcums in Gelatinekapseln abgefüllt.

| | |
|---|---|
| Kapselfüllgewicht | 450 mg |
| Wirkstoffgehalt | 250 mg |

**Patentansprüche**

1. Imidazolidinderivate der allgemeinen Formel

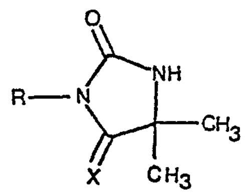

I

in der X Sauerstoff oder Imino und R eine der Gruppen

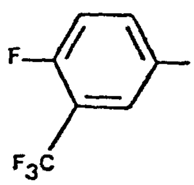 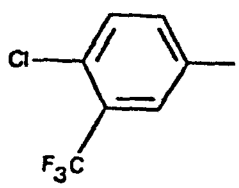 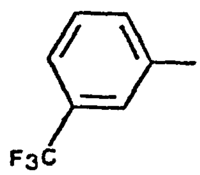 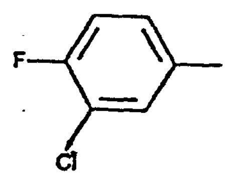 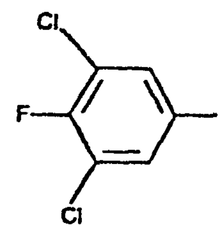

darstellt.

2. Imidazolidinderivate gemäss Anspruch 1, dadurch gekennzeichnet, dass R eine der Gruppen

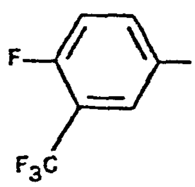 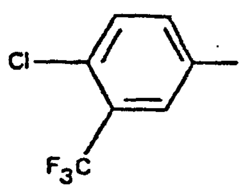 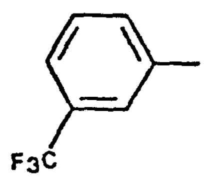 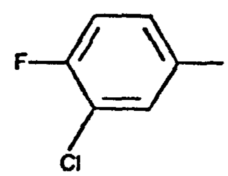

darstellt.

3. Imidazolidinderivate gemäss Anspruch 1, dadurch gekennzeichnet, dass X Sauerstoff darstellt.
4. Imidazolidinderivate gemäss Anspruch 2, dadurch gekennzeichnet, dass X Sauerstoff darstellt.
5. 3-(3-Chlor-4-fluor-phenyl)-5,5-dimethyl-hydantoin.
6. 3-(3-Trifluormethyl-4-fluor-phenyl)-5,5-dimethylhydantoin.
7. 1-(3-Trifluormethyl-4-fluor-phenyl)-5-imino-4,4-dimethyl-2-imidazolidinon.
8. Verfahren zur Herstellung der Imidazolidinderivate gemäss einem der Ansprüche 1—7, dadurch gekennzeichnet, dass man

a) zur Herstellung einer Verbindung der Formel I, in der X Sauerstoff darstellt, eine Verbindung der allgemeinen Formel

$$R{-}NH{-}CO{-}NH{-}\overset{\displaystyle CH_3}{\underset{\displaystyle CH_3}{C}}{-}COY \qquad II$$

in der R die obige Bedeutung hat und Y die Gruppe $-OR^1$ oder $-NR^2R^3$ darstellt, worin $R^1$ Wasserstoff oder niederes Alkyl und $R^2$ und $R^3$ beide niederes Alkyl darstellen,
cyclisiert, oder dass man

b) zur Herstellung einer Verbindung der Formel I, in der X Imino bedeutet, eine Verbindung der allgemeinen Formel

R—NCO III

in der R die obige Bedeutung hat,
unter wasserfreien Bedingungen mit $\alpha$-Amino-isobutyronitril umsetzt, oder dass man

c) zur Herstellung einer Verbindung der Formel I, in der X Sauerstoff darstellt, eine Verbindung der allgemeinen Formel

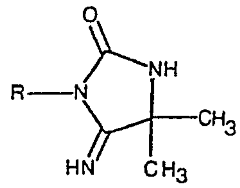

Ia

in der R die obige Bedeutung hat,
einer Hydrolyse unterwirft.

9. Zwischenprodukt der allgemeinen Formel

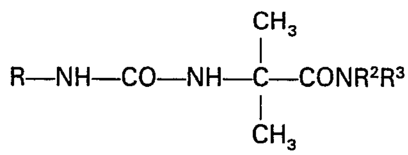

IIa

in der R eine der Gruppen

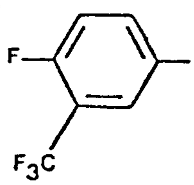 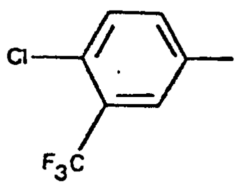 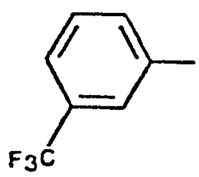 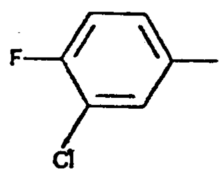 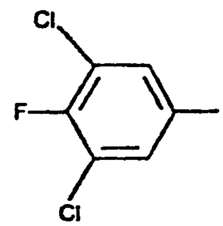

und $R^2$ und $R^3$ beide niederes Alkyl darstellen,
wenn es in einem Verfahren gemäss Anspruch 8 verwendet wird.

10. Pharmazeutisches Präparat, gekennzeichnet durch einen Gehalt an einem Imidazolidinderivat gemäss einem der Ansprüche 1—7.

11. Imidazolidinderivate gemäss einem der Ansprüche 1—7 zur Verwendung bei der Bekämpfung von Krankheiten, die mit einer erhöhten androgenen Aktivität in Verbindung stehen.

12. Imidazolidinderivate gemäss einem der Ansprüche 2 und 4—7 zur Verwendung bei der Bekämpfung bzw. Verhütung von Bilharziose.

**Claims**

1. Imidazolidine derivatives of the general formula

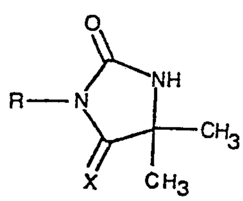

I

in which X represents oxygen or imino and R represents one of the groups

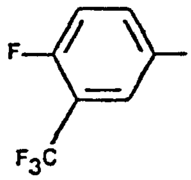 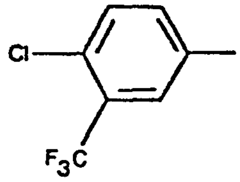 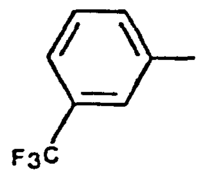 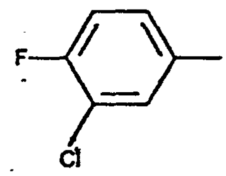 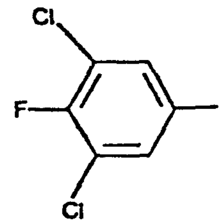

2. Imidazolidine derivatives in accordance with claim 1, characterised in that R represents one of the groups

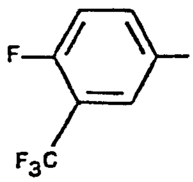 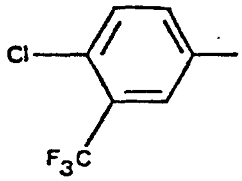 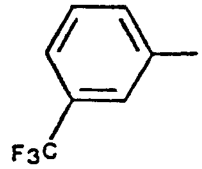 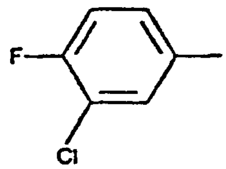

3. Imidazolidine derivatives in accordance with claim 1, characterised in that X represents oxygen.
4. Imidazolidine derivatives in accordance with claim 2, characterised in that X represents oxygen.
5. 3-(3-Chloro-4-fluoro-phenyl)-5,5-dimethyl-hydantoin.
6. 3-(3-Trifluoromethyl-4-fluoro-phenyl)-5,5-dimethyl-hydantoin.
7. 1-(3-Trifluoromethyl-4-fluoro-phenyl)-5-imino-4,4-dimethyl-2-imidazolidinone.
8. Process for the manufacture of the imidazolidine derivatives in accordance with one of claims 1—7, characterised in that

a) for the manufacture of a compound of formula I in which X represents oxygen, a compound of the general formula

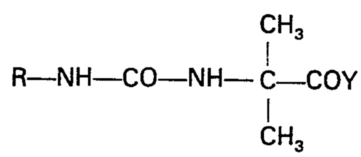 II

in which R has the above significance and Y represents the group —OR[1] or —NR[2]R[3], wherein R[1] represents hydrogen or lower alkyl and R[2] and R[3] both represent lower alkyl, is cyclised, or in that

b) for the manufacture of a compound of formula I in which X signifies imino, a compound of the general formula

R—NCO III

in which R has the above significance is reacted under anhydrous conditions with $\alpha$-amino-isobutyronitrile, or in that

c) for the manufacture of a compound of formula I in which X represents oxygen, a compound of the general formula

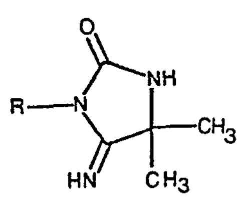 Ia

in which R has the above significance, is subjected to a hydrolysis.

9. Intermediate of the general formula

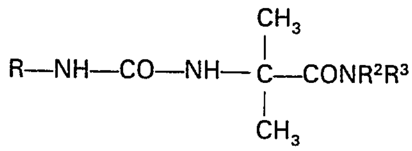

IIa

in which R represents one of the groups

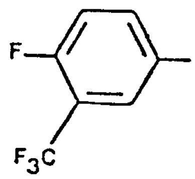
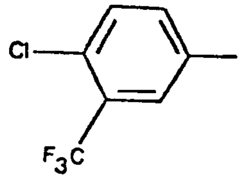
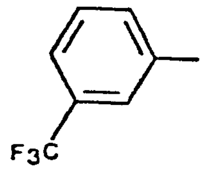
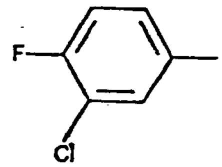
,
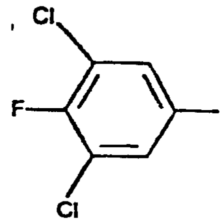

and $R^2$ and $R^3$ both represent lower alkyl, when used in a process in accordance with claim 8.

10. Pharmaceutical preparation, characterised in that it contains an imidazolidine derivative in accordance with one of claims 1—7.

11. Imidazolidine derivatives in accordance with one of claims 1—7 for use in control of illnesses which are associated with an increased androgenic activity.

12. Imidazolidine derivatives in accordance with one of claims 2 and 4—7 for use in the control or prevention of bilharziasis.

**Revendications**

1. Dérivés d'imidazolidine de formule générale

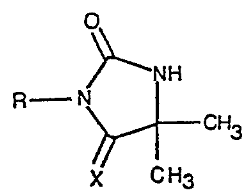

I

où X représente un oxygène ou un imino et R l'un des groupes

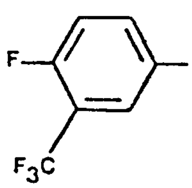
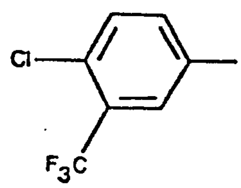
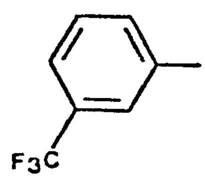
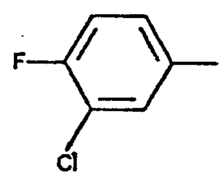
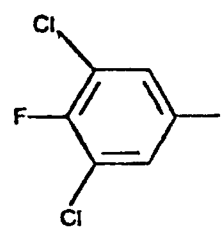

2. Dérivés d'imidazolidine selon la revendication 1, caractérisés en ce que R représente l'un des groupes

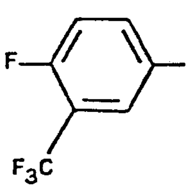
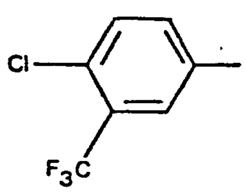
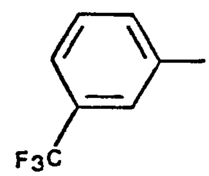
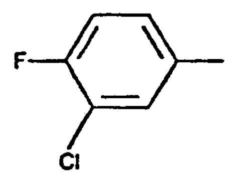

3. Dérivés d'imidazolidine selon la revendication 1, caractérisés en ce que X représente un oxygène.

4. Dérivés d'imidazolidine selon la revendication 2, caractérisés en ce que X représente un oxygène.

5. La 3-(3-Chloro-4-fluoro-phényl)-5,5-diméthyl-hydantoïne.

6. La 3-(3-Trifluorométhyl-4-fluoro-phènyl)-5,5-diméthyl-hydantoïne.

7. La 1-(3-Trifluorométhyl-4-fluoro-phényl)-5-imino-4,4-diméthyl-2-imidazolidinone.

8. Procédé de préparation des dérivés d'imidazolidine selon l'une des revendications 1 à 7, caractérisé en ce que

a) Pour préparer un composé de formule I où X représente un oxygène, on cyclise un composé de formule générale

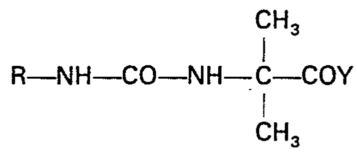
II

où R a la signification donnée ci-dessus et où Y représente le groupe —$OR^1$ ou —$NR^2R^3$, où $R^1$ représente un hydrogène ou un alcoyle inférieur et où $R^2$ et $R^3$ représentent tous deux un alcoyle inférieur, ou

b) Pour préparer un composé de formule I où X représente un imino, on fait réagir un composé de formule générale

R—NCO III

où R a la signification donnée ci-dessus, dans des conditions anhydres avec de l'alpha-amino-isobutyronitrile, ou

c) Pour préparer un composé de formule I où X représente un oxygène, ou soumet à une hydrolyse un composé de formule générale

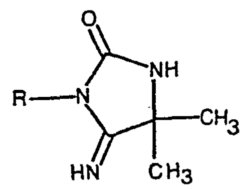
Ia

où R a la signification donnée ci-dessus.

9. Produit intermédiaire de formule générale

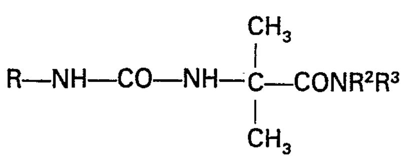
IIa

où R représente l'un des groupes

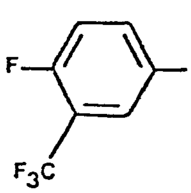
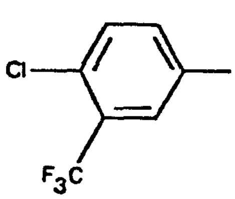
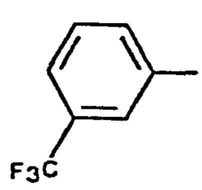
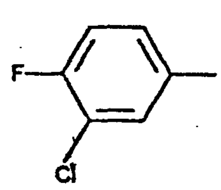
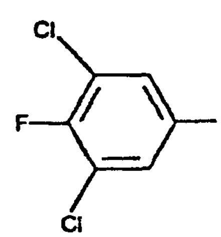

et où $R^2$ et $R^3$ représentent tous deux un alcoyle inférieur, lorsqu'on l'applique dans un procédé selon la revendication 8.

10. Préparation pharmaceutique caractérisée en ce qu'elle contient un dérivé d'imidazolidine selon l'une des revendications 1 à 7.

11. Dérivés d'imidazolidine selon l'une des revendications 1 à 7 destinés à l'application dans la lutte contre les maladies liées à une augmentation d'activité androgène.

12. Dérivés d'imidazolidine selon l'une des revendications 2 et 4 à 7 aux fins d'application dans la lutte ou la prévention de la bilharziose.